# EUROPEAN PATENT SPECIFICATION

(11) **EP 1 954 241 B1**
(45) Date of publication and mention of the grant of the patent: **29.02.2012**
(21) Application number: 06838427.0
(22) Date of filing: 27.11.2006
(51) Int. Cl.: A61K 9/50, A61K 9/00, A61K 9/20, A61K 31/137, A61K 31/42

(54) **SUSTAINED-RELEASE FORMULATION OF ZONISAMIDE**
ZONISAMID-FORMULIERUNG MIT VERZÖGERTER FREISETZUNG
FORMULATION DE ZONISAMIDE A LIBERATION PROLONGEE

(30) Priority: 28.11.2005 US 740034 P; 19.07.2006 US 832110 P; 04.08.2006 US 835564 P
(43) Date of publication of application: 13.08.2008
(73) Proprietor: Orexigen Therapeutics, Inc., San Diego, CA 92130 (US)
(72) Inventor: MCKINNEY, Anthony A., San Diego, CA 92130 (US); TOLLEFSON, Gary, Indianapolis, IN 46236 (US); YAU, Simon, Watchung, NJ 07069 (US); VLADYKA, Ron, Somerset, NJ 08873 (US); SOLTERO, Rick, Holly Springs, NC 27540 (US)
(74) Representative: Carpintero Lopez, Francisco
(86) International application number: PCT/US2006/045445
(87) International publication number: WO 2007/062228

(56) References cited:
- WO-A-03/097046
- WO-A-2004/024096
- WO-A-2004/050020
- WO-A-2004/100992
- US-A1- 2004 158 194

## Description

### Field of the Invention

The present embodiments are directed to novel formulations of zonisamide, including sustained-release formulations.

### Description of the Related Art

Zonisamide is a sodium channel blocker useful in the treatment of epilepsy and is marketed as an anticonvulsant. It is chemically known as 1,2-benzisoxazole-3-methanesulfonamide. ZONEGRAN^{®} zonisamide capsules, available commercially from Eisai, Inc., are immediate-release capsules designed for oral administration of one to four capsules once per day to provide a daily dose of 100 to 400 mg. Peak plasma concentrations (Cₘₐₓ) of zonisamide are generally achieved at between 2-6 hours after administration of the immediate-release form. Zonisamide, has a half-life (t_{½}) in plasma of about 63 to 69 hours, which allows twice-daily or even once daily dosing, *see* Leppik IE., "Zonisamide: chemistry, mechanism of action, and pharmacokinetics," Seizure 2004 Dec; 13 Suppl 1:S5-9; discussion S10. Those skilled in the art have thus far not been particularly motivated to prepare controlled-release zonisamide formulations because of the relatively long time to achieve Cₘₐₓ using the immediate-release form and the relatively long half-life of zonisamide in plasma.

The international patent application W02003/097046 discloses a sustained released formulations such as tablet or capsule comprising a combination of zonisamide and bupropion for the effective treatment for obesity, control of appetite, weight loss.

The international patent applications W02004/024096 and W02004/100992 disclose tablets, pills or capsules comprising sustained or controlled release forms for the prolonged administration of zonisamide. The formulations are orally administered and contain conventional excipients such as binders, disintegrants and carriers which can be selected from the group of for example polyvinylpyrrolidone, microcrystalline cellulose, calcium phosphate, hydroxypropylmethylcellulose,

### SUMMARY

The present invention relates to sustained release pharmaceutical formulations comprising zonisamide and at least 5% of a retardant excipient.

### SUMMARY

The retardant excipient is configured to modify the dissolution profile of the sustained-release zonisamide.

These and other embodiments are described in greater detail below.

### BRIEF DESCRIPTION OF THE FIGURES

Other aspects of the disclosure will be readily apparent from the description below and the appended drawings, in which:

Figure 1 illustrates dose-normalized total serum concentration time profiles for zonisamide immediate-release and zonisamide sustained-release slow formulations following a single oral dose as a function of time.

Figure 2 illustrates a plot of Cₘₐₓ of total serum zonisamide as a function of time.

Figure 3 illustrates a plot of Cₘₐₓ of whole blood zonisamide as a function of time.

### DETAILED DESCRIPTION OF THE PREFERRED EMBODIMENTS

Various embodiments provide pharmaceutical formulations that comprise sustained-release zonisamide. Such formulations can be configured in various ways and in a variety of dosage forms, such as tablets and beads, to modify the release of the zonisamide. Sustained-release zonisamide pharmaceutical formulations according to the invention contain retardant excipients (also referred to as release modifiers) and optionally fillers at are selected and incorporated into the formulation in such a way as to slow the dissolution rate of the formulation (and thereby slow the dissolution and/or release of the zonisamide) under in vivo conditions as compared to an otherwise comparable immediate-release formulation. Thus, a "comparable" immediate-release formulation is one that is substantially identical to the sustained-release formulation, except that that it is configured to provide immediate-release instead of controlled-release under substantially identical conditions.

The term "immediate-release" is used herein to specify a formulation that is not configured to alter the dissolution profile of the active ingredient (e.g., zonisamide). For example, an immediate-release pharmaceutical formulation may be a pharmaceutical formulation that does not contain ingredients that have been included for the purpose of altering the dissolution profile. An immediate-release formulation thus includes drug formulations that take less than 30 minutes for substantially complete dissolution of the drug in a standard dissolution test. A "standard dissolution test," as that term is used herein, is a test conducted according to United States Pharmacopeia 24th edition (2000) (USP 24), pp. 1941-1943, using Apparatus 2 described therein at a spindle rotation speed of 100 rpm and a dissolution medium of water, at 37°C, or other test conditions substantially equivalent thereto. The term "controlled-release" is used herein in its ordinary sense and thus includes pharmaceutical formulations that are combined with ingredients to alter their dissolution profile. A "sustained-release" formulation is a type of controlled-release formulation, wherein ingredients have been added to a pharmaceutical formulation such that the dissolution profile of the active ingredient is extended over a longer period of time than that of an otherwise comparable immediate-release formulation. A controlled-release formulation thus includes drug formulations that take 30 minutes or longer for substantially complete dissolution of the drug in a standard dissolution test, conditions which are representative of the in vivo release profile.

The term "orally deliverable" is used herein in its ordinary sense and thus includes drug formulations suitable for oral, including peroral and intra-oral (e.g., sublingual or buccal) administration. Preferred compositions are adapted primarily for peroral administration, e.g., for swallowing. Examples of preferred orally deliverable compositions include discrete solid articles such as tablets and capsules, which are typically swallowed whole or broken, with the aid of water or other drinkable fluid.

The term "therapeutically effective amount" is used herein in its ordinary sense and thus includes daily dosage amounts of a drug or drug combination that, when administered as part of a regimen, provides therapeutic benefit in the treatment of a condition or disorder for which the drug or drug combination is indicated. For example, in some preferred embodiments, amounts per dose of zonisamide are likely to be found in a range from about 10 mg to about 400 mg, in more preferred embodiments amounts per dose are found in a range of about 50 mg to about 100 mg.

The term "pharmaceutically acceptable salt" is used herein in its ordinary sense and thus includes a formulation of a compound that does not cause significant irritation to an organism to which it is administered and does not abrogate the biological activity and properties of the compound. Pharmaceutical salts can be obtained by reacting a compound of the present disclosure with an inorganic acid such as hydrochloric acid, hydrobromic acid, sulfuric acid, nitric acid, phosphoric acid, methanesulfonic acid, ethanesulfonic acid, p-toluenesulfonic acid, salicylic acid and the like. Pharmaceutical salts can also be obtained by reacting a compound of the present disclosure with a base to form a salt such as ammonium salt, an alkali metal salt such as a sodium or a potassium salt, an alkaline earth metal salt such as a calcium or a magnesium salt, a salt of an organic base such as dicyclohexylamine, N-methyl-D-glucamine, tris(hydroxymethyl) methylamine and salts thereof with amino acids such as arginine, lysine and the like. Unless the context dictates otherwise, reference herein to a particular compound will be understood to include such salt forms.

The term in vivo "absorption" is used herein in its ordinary sense and thus includes reference to the percentage of zonisamide that enters the bloodstream, as conventionally calculated from data of a standard pharmacokinetic (PK) study involving oral administration of a single dose of zonisamide. It will be understood that PK data are subject to the usual variation seen in biological data, thus the absorption percentages specified herein are means from a population, typically at least about 20 in number, of individual healthy adults in accordance with standard statistical practice.

A "subject" herein is an animal of any species, preferably mammalian, most preferably human. Conditions and disorders in a subject for which a particular drug or compound (such as zonisamide) is said herein to be "indicated" are not restricted to conditions and disorders for which that drug or compound has been expressly approved by a regulatory authority, but also include other conditions and disorders known or reasonably believed by a physician to be amenable to treatment with that drug or compound. "Treatment" herein embraces prophylactic treatment unless the context requires otherwise.

The sustained-release zonisamide pharmaceutical formulation comprises one or more retardant excipients. In this context, the term "retardant" excipient is used herein in its ordinary sense and thus includes an excipient that is configured (e.g., incorporated into the formulation) in such a way as to control a dissolution profile of the drug, e.g., slow the dissolution of the zonisamide in a standard dissolution test, as compared to an otherwise comparable pharmaceutical formulation that does not contain the retardant excipient. Examples of pharmaceutically acceptable retardant excipients include hydroxypropyl methylcellulose (HPMC), hydroxyethylcellulose, hydroxypropylcellulose (HPC), methylcellulose, ethylcellulose, cellulose acetate butyrate, cellulose acetate phthalate, hydroxypropylmethyl cellulose phthalate, microcrystalline cellulose, corn starch, polyethylene oxide, polyvinyl alcohol (PVA), polyvinylpyrrolidone (PVP), cross-linked PVP, polyvinyl acetate phthalate, polyethylene glycol, zein, poly-DL-lactide-co-glycolide, dicalcium phosphate, calcium sulfate, and mixtures thereof. In some embodiments the retardant excipient comprises a sustained-release polymer, e.g., at least one of hydroxypropyl methylcellulose (HPMC), hydroxyethylcellulose, hydroxypropylcellulose (HPC), methylcellulose, ethylcellulose, cellulose acetate butyrate, cellulose acetate phthalate, hydroxypropylmethyl cellulose phthalate, microcrystalline cellulose, corn starch, polyethylene oxide, polyvinyl alcohol (PVA), polyvinylpyrrolidone (PVP), cross-linked PVP, polyvinyl acetate phthalate, polyethylene glycol, zein, poly-DL-lactide-co-glycolide, and mixtures thereof. Retardant excipients may be referred to herein as release modifiers.

There are various ways that an excipient can be configured to control a dissolution profile of a sustained-release formulation. For example, the excipient can be intimately mixed with the drug (e.g., zonisamide) in an amount effective for controlling release of the drug from the pharmaceutical formulation. Such a mixture can be in various forms, e.g., a dry mixture, a wet mixture, beads, etc., and may be formed in various ways. The resulting mixture can then be formed into the desired dosage form, e.g., tablet or capsule. The sustained-release zonisamide pharmaceutical formulation comprises at least about 5 weight %, preferably at least about 10 weight %, of the retardant excipient(s). Effective amounts of retardant excipient(s) for controlling release may be determined by routine experimentation informed by the guidance provided herein.

Various dissolution characteristics of the dissolution profile of the sustained-release zonisamide pharmaceutical formulation can be controlled by appropriate configuration of the retardant excipient incorporated therein. Preferably, the dissolution profile comprises a dissolution rate that is slower than a dissolution rate of a comparable immediate-release zonisamide formulation. For example, in some embodiments, the pharmaceutical formulation comprises zonisamide and at least one retardant excipient configured to control an *in vitro* release profile within the following ranges:

| **Hour** | **% Released** |
|---|---|
| 1 | 0 - 40% |
| 4 | 10 - 60% |
| 8 | 20 - 80% |
| 12 | >= 70% |

The *in vitro* release profile may be estimated by dissolution measurements in water at 37° C. For example, a preferred dissolution profile comprises at least one dissolution characteristic selected from:
(a) less than about 70% of zonisamide in the sustained-release zonisamide is dissolved within a first hour in a standard dissolution test,
(b) less than about 40% of the zonisamide in the sustained-release zonisamide is dissolved within a first hour in a standard dissolution test,
(c) less than about 30% of the zonisamide in the sustained-release zonisamide is dissolved within a first hour in a standard dissolution test,
(d) less than about 75% of the zonisamide in the sustained-release zonisamide is dissolved within a second hour in a standard dissolution test,
(e) less than about 55% of the zonisamide in the sustained-release zonisamide is dissolved within a second hour in a standard dissolution test, and
(f) less than about 35% of the zonisamide in the sustained-release zonisamide is dissolved within a second hour in a standard dissolution test.

In an embodiment, the sustained-release pharmaceutical formulation comprises zonisamide and at least one retardant excipient configured to provide, upon administration to a patient, an average free serum zonisamide Cₘₐₓ value that is less than (e.g., at least about 5% less than) the average free serum zonisamide Cₘₐₓ value of a comparable immediate-release zonisamide under comparable conditions. For example, the retardant excipient can be configured to control an *in vivo* free zonisamide serum profile wherein there is greater zonisamide bioavailabilty, as indicted by an area under the serum concentration curve at steady state that is substantially equal to or greater than a conventional immediate-release zonisamide formulation at the same dose, and a lower Cₘₐₓ at steady state than a conventional immediate-release zonisamide formulation at the same dose.

Sustained-release zonisamide pharmaceutical formulation as described herein may be formulated to be useful for oral administration under dosage schedules in the range of once or twice daily to once every two to seven days, to a subject having a condition or disorder for which the administration of zonisamide is indicated. Thus, in some embodiments a pharmaceutical formulation comprises a controlled dosage form suitable for daily or weekly administration of zonisamide.

Certain sustained-release zonisamide formulations exhibit one or more surprising and unexpected features and benefits. For example, sustained-release dosage forms are typically sought to enable longer time intervals between dosing of a drug having a short half-life in plasma, due for example to rapid metabolism, excretion or other routes of depletion. Among drugs used to treat Parkinson's disease, levodopa is a well-known example, having a short elimination half-life (T_{½}) of about 1.5 hours. *See* Colosimo & De Michele, European Journal of Neurology 6 (1), 1-21 (1999). In contrast, zonisamide has a t_{½} of about 63 hours in plasma to about 105 hours in erythrocytes, depending on the particular study, and would not on this basis be expected to require special attention to formulation to enable once-daily dosing.

Zonisamide has high solubility in aqueous acid (about 200 mg/ml at 20-25°C). Highly water-soluble drugs are typically difficult to formulate in sustained-release form because of the tendency of the drug to rapidly leach out of the dosage form upon exposure to an aqueous medium such as gastrointestinal fluid.

Sustained-release zonisamide dosage forms having very different in vitro release profiles, as characterized by standard parameters such as percentage released in 1, 4, 8 and 12 hours, can, as demonstrated herein, have in vivo PK profiles that are similar to immediate-release dosage forms, but have in vivo PK profiles that differ in very meaningful ways at steady state. This is especially unexpected as one ordinarily expects a slowly-released drug to have lower bioavailability than an immediately released drug. It is surprisingly found that preferred sustained-release dosage forms of zonsiamide as described herein have at least the same bioavailability and in some cases increased bioavailability compared to the immediate-release dosage form at steady state. It is surprisingly found as well that preferred sustained-release dosage forms of zonsiamide have lower Cₘₐₓ and lower serum free zonisamide fraction concentrations compared to the immediate-release dosage form while the Area Under the Curve (AUC) is comparable to the immediate-release dosage form.

In certain embodiments, the sustained-release zonisamide pharmaceutical formulation, when administered once or twice daily, exhibits a bioavailability, as expressed conventionally by AUC₀₋₂₄ or AUC_{0-∞}, which is substantially equivalent to the same daily dose of an immediate-release zonisamide dihydrochloride reference formulation. In the present context, "substantially equivalent" means that the bioavailability of such a preferred composition is about 0.8 to about 1.25 times that of the reference formulation.

In an embodiment, a method of treatment comprises administering a sustained-release zonisamide pharmaceutical formulation as described herein to a patient in need thereof. In a preferred embodiment, the patient experiences a reduced risk of an adverse event associated with administering a comparable dosage of an immediate-release zonisamide For example, in some embodiments, a sustained-release zonisamide pharmaceutical formulation having in vitro release and/or in vivo PK parameters as described herein is advantageous in having reduced potential to cause undesirable adverse events that may be related to a combination of high Cₘₐₓ and short Tₘₐₓ, in comparison with other once-daily dosage forms (such as immediate-release forms). In some embodiments, an incidence of adverse events is no greater than with an immediate-release dosage form. More preferably, the incidence of adverse events is significantly lower than with such an immediate-release regimen. Preferably these advantages become more pronounced with increases in daily dosage during the initiation and/or course of zonisamide therapy.

In some embodiments, the sustained-release zonisamide pharmaceutical formulation is formed into capsules, tablets or other solid dosage forms suitable for oral administration. In preferred embodiments, the sustained-release zonisamide pharmaceutical formulation is formulated as a discrete solid dosage unit such as a tablet or capsule, wherein the zonisamide or salt thereof is present therein as particles, and is formulated together with one or more pharmaceutically acceptable excipients. In some embodiments the excipients are retardant excipients selected at least in part to provide a release profile and/or PK profile consistent with the desired profiles described herein.

In some embodiments the particular solid dosage form selected is not critical so long as it achieves a release and/or PK profile as defined herein for the particular sustained-release formulation. In some embodiments the profile is achieved using one or more retardant excipients or release modifiers. In some embodiments release modifiers suitable for use include a polymer matrix with which and/or in which the zonisamide is dispersed; a release-controlling layer or coating surrounding the whole dosage unit or zonisamide-containing particles, granules, beads or zones within the dosage unit; and an osmotic pump.

Sustained-release zonisamide pharmaceutical formulations can be configured in a variety of dosage forms, such as tablets and beads; can contain a variety of fillers and excipients, such as retardant excipients (also referred to a release modifiers); and may be made in a variety of ways. Those skilled in the art may determine the appropriate configuration by routine experimentation guided by the descriptions provided herein.

Sustained-release zonisamide pharmaceutical formulations may contain fillers. Examples of suitable fillers include, but are not limited to, METHOCEL^{®} methylcellulose, hydroxypropyl methylcellulose (HPMC), hydroxypropylcellulose (HPC), corn starch, polyvinyl alcohol (PVA), polyvinylpyrrolidone (PVP), cross-linked PVP, and the like.

Sustained-release zonisamide pharmaceutical formulations may contain excipients. Examples of suitable excipients include, but are not limited to, acetyltriethyl citrate (ATEC), acetyltri-n-butyl citrate (ATBC), aspartame, lactose, alginates, calcium carbonate, carbopol, carrageenan, cellulose, cellulose acetate phthalate, croscarmellose sodium, crospovidone, dextrose, dibutyl sebacate, ethylcellulose, fructose, gellan gum, glyceryl behenate, guar gum, lactose, lauryl lactate, low-substituted hydroxypryopl cellulose (L-HPC), magnesium stearate, maltodextrin, maltose, mannitol, methylcellulose, microcrystalline cellulose, methacrylate, sodium carboxymethylcellulose, polyvinyl acetate phthalate (PVAP), povidone, shellac, sodium starch glycolate, sorbitol, starch, sucrose, triacetin, triethylcitrate, vegetable based fatty acid, xanthan gum, xylitol, and the like.

In preferred embodiments, the sustained-release zonisamide pharmaceutical formulation comprises zonisamide, methylcellulose and microcrystalline cellulose. In some embodiments, the formulation comprises, for example, from about 30%, 40%, or 50%, to about 80% or 90% zonisamide by weight. In some embodiments, the formulation comprises about 0.1%, 0.5%, 1%, 3%, 5%, 10% or 20% zonisamide by weight. Preferably, the zonisamide is present at a percentage of about 55%, 60%, 65%, or 70% by weight. In other preferred embodiments, the formulation comprises about 95% zonisamide.

The balance of ingredients in the sustained-release zonisamide pharmaceutical formulation can be chosen, for example, from modified polysaccharides such as, for example, methylcellulose (MC) and microcrystalline cellulose (MCC). In some embodiments, the formulation comprises between about 3% to about 99.9% microcrystalline cellulose by weight. In certain embodiments, the formulation comprises about 3% MCC. In other embodiments, the formulation comprises about 5% MCC. In further embodiments, the formulation comprises about 10% MCC. In yet other embodiments, the formulation comprises about 30% MCC. In further embodiments, the formulation comprises about 50% MCC.

In some embodiments, the sustained-release zonisamide pharmaceutical formulation comprises about 0% to about 40% MC. In certain embodiments, the formulation comprises about 3% MC. In other embodiments, the formulation comprises about 5% MC. In further embodiments, the formulation comprises about 10% MC. In yet other embodiments, the formulation comprises about 30% MC. In further embodiments, the formulation comprises about 40% MC. In some embodiments, the formulation comprises about 95% zonisamide and the remaining 5% is divided between MC and MCC.

The dissolution rate of the sustained-release zonisamide pharmaceutical formulation determines how quickly zonisamide becomes available for absorption into the blood stream and therefore controls the bioavailability of zonisamide. Dissolution rate is dependent on the size and the composition of the dosage form. In some embodiments, the dissolution rate of the zonisamide formulation can be by changed by altering the additional components of the formulation. Disintegrants, such as starch or corn starch, or crosslinked PVPs, can be used to increase solubility when desired. Solubilizers can also be used to increase the solubility of the zonisamide formulations. In some embodiments alternative binders, such as hydroxypropylmethyl cellulose (HPMC), hydroxypropyl cellulose (HPC), methyl cellulose (MC), PVP, gums, xanthine, and the like, can be used to increase the dissolution rate.

In some embodiments the dissolution rate of the formulation can be decreased by adding components that make the formulation more hydrophobic. For example, addition of polymers such as ethylcelluloses, wax, magnesium stearate, and the like decreases the dissolution rate.

In some embodiments, the dissolution rate of the sustained-release zonisamide pharmaceutical formulation is such that about 25% of the zonisamide in the dosage form is dissolved within the first hour, about 60% of the zonisamide is dissolved within the first 6 hours, about 80% of the zonisamide is dissolved within the first 9 hours, and substantially all of the zonisamide is dissolved within the first 12 hours. In other embodiments, the dissolution rate of the sustained-release zonisamide pharmaceutical formulation is such that about 35% of the zonisamide in the dosage form is dissolved within the first hour, about 85% of the zonisamide is dissolved within the first 6 hours, and substantially all of the zonisamide is dissolved within the first 9 hours. In yet other embodiments, the dissolution rate of the sustained-release zonisamide pharmaceutical formulation in the dosage form is such that about 45% of the zonisamide in the beads is dissolved within the first hour, and substantially all of the zonisamide is dissolved within the first 6 hours.

The dissolution rate of the formulation can also be slowed by coating the dosage form. Examples of coatings include enteric coatings, sustained-release polymers, and the like.

The sustained-release zonisamide pharmaceutical formulation can take about, for example, from 2, 4, 6, or 8 hours to about 15, 20, or 25 hours to dissolve. Preferably, the formulation has a dissolution rate of from about 3, 4, 5, or 6 to about 8, 9, or 10 hours.

Another embodiment provides a method of preparing sustained-release zonisamide pharmaceutical formulation. The method comprises mixing zonisamide with an excipient and/or filler to form a mixture, and forming a suitable dosage form (e.g., tablet, bead, etc.) from the mixture. In some embodiments, the method of preparing the formulation further comprises adding another excipient and/or filler to the mixture prior to forming the dosage form. The filler and excipient are as described herein. In an embodiment, the zonisamide is mixed with the filler and/or excipient to form a wet mixture. The wet mixture can then be formed into particles or beads, which can then be dried. The dried product can then be tableted or placed into a gelatin capsule for oral delivery.

In an embodiment, the sustained-release zonisamide pharmaceutical formulation is in the form of beads. In some embodiments, the beads comprise zonisamide and a filler. In other embodiments, the beads further comprise an excipient. In some embodiment, the filler and/or the excipient are in polymeric form.

As used herein, "beads" can be, for example, spheres, pellets, microspheres, particles, microparticles, granules, and the like. The beads can have any desired shape. The shape can be, for example, spherical, substantially spherical, rod-like, cylindrical, oval, elliptical, granular, and the like. The size and shape of the bead can be modified, if desired, to alter dissolution rates. The beads may be coated or may be uncoated. The beads may be formed into a capsule for oral delivery, a tablet, or any other desired solid oral dosage form, with or without other ingredients.

In an embodiment, a pharmaceutical formulation comprises a bead that comprises sustained-release zonisamide and a filler. In some embodiments the bead further comprises an excipient. In some embodiments the filler is a polymer. In some embodiments the excipient is a polymer. In some embodiments the filler is selected from the group consisting of methylcellulose, hydroxypropyl methylcellulose (HPMC), hydroxypropylcellulose (HPC), corn starch, polyvinyl alcohol (PVA), polyvinylpyrrolidone (PVP), and cross-linked PVP. In some embodiments the excipient is selected from the group consisting of acetyltriethyl citrate (ATEC), acetyltri-n-butyl citrate (ATBC), aspartame, lactose, alginates, calcium carbonate, carbopol, carrageenan, cellulose, cellulose acetate phthalate, croscarmellose sodium, crospovidone, dextrose, dibutyl sebacate, ethylcellulose, fructose, gellan gum, glyceryl behenate, guar gum, lactose, lauryl lactate, low-substituted hydroxypropyl cellulose (L-HPC), magnesium stearate, maltodextrin, maltose, mannitol, methylcellulose, microcrystalline cellulose, methacrylate, sodium carboxymethylcellulose, polyvinyl acetate phathalate (PVAP), povidone, shellac, sodium starch glycolate, sorbitol, starch, sucrose, triacetin, triethylcitrate, vegetable based fatty acid, xanthan gum, and xylitol. In some embodiments the bead comprises zonisamide, methylcellulose and microcrystalline cellulose. In some embodiments the bead comprises from about 0.1% to about 95% zonisamide by weight. In some embodiments the bead comprises between about 3% to about 99.9% microcrystalline cellulose by weight. In some embodiments the bead comprises about 0% to about 40% methylcellulose by weight.

In some embodiments the beads can advantageously be formed from a wet mixture using any suitable apparatus. Preferably, an extrusion device is used. The wet mixture can be extruded to form "spaghetti-like" strands. These can be cut, preferably as they are being extruded, to form pellets of a desired size. The wet cylindrical pellets can, in turn, be placed into a "spheronizer" that forms them into generally spherical shapes. The spheronizer comprises a rotating plate or other rotating mechanism onto or into which the pellets are introduced and maintained for a sufficient time to generate beads of a desired spherical shape. These spheres can then be collected and dried by any suitable means. Preferably, the beads are dried using a fluid bed drying process. Other suitable means for drying the beads, as will be known by those in the art, can be used.

The spheronizer is preferably fitted with a screen having holes of a specified size, such as 16 mesh, 18 mesh, 20 mesh, 25 mesh, or other sizes. The screen causes beads of certain diameter to leave the spheronizer, thereby generating beads of relatively uniform diameter. In some embodiments, the zonisamide bead size can range, for example, from a range of about 10, 50, or 100 µm to about 700, 900, or 1,000 µm. In other embodiments, the bead size is from about 150, 200, 250 µm, to about 300, 400, or 500 µm. In yet other embodiments, the bead size is about 200 µm. In some embodiments, the preferred bead diameter is between 0.71 mm and 1.17 mm.

The spheronizer speed also has an effect on the bead size. Faster spheronizer rotation speeds result in smaller beads. In some embodiments, the spheronizer speed is between about 1 and about 900 rpm. In further embodiments, the spheronizer speed is between about 10 and about 800 rpm.

In another embodiment a method of preparing beads comprising zonisamide in a sustained-release formulation, comprises mixing zonisamide with a filler to form a mixture and forming beads from the mixture. In some embodiments the method further comprises adding an excipient to the mixture prior to forming the beads. In some embodiments the method further comprises forming strands from the mixture, cutting the strand to form "pellets and forming generally spherical shaped beads from the pellets using a spheronizer. In some embodiments the diameter of the beads is between 0.71 mm and 1.17 mm.

The inventors have found that sustained-release zonisamide pharmaceutical formulations dissolve more slowly than the immediate-release formulations that are representative of currently-marketed ZONEGRAN^{®} zonisamide, thus providing a novel sustained-release form of zonisamide that can slow dissolution by up to about 10 hours. This finding was surprising, as it was expected that the presence of hydrophilic cellulosic compounds in the formulation would cause the composition to crumble in water and would be likely to increase the dissolution rate. In some embodiments other factors, such as a higher drug loading of zonisamide in the beads contribute to the unexpected finding that the formulation of the embodiments disclosed herein results in a slower dissolution rate.

Peak plasma concentrations of zonisamide are typically achieved between 2-6 hours after administration of the immediate-release form. However, the pharmacodynamics of the drug are complicated by the fact that the majority of the zonisamide in the blood is bound to erythrocytes. Adverse events of zonisamide have been reported to include headache, nausea and vomiting, and sleepiness/sedation which are dose related. Patients may complain about the adverse events and often discontinue using the product.

Patient compliance with a zonisamide treatment is much improved by administration of a sustained-release formulation that results in reaching a steady state concentration substantially equivalent to the immediate-release zonisamide capsule, but with a lower effective dose (resulting in a lower Cₘₐₓ). An important feature of a preferred sustained-release zonisamide formulation is the more effective control of free fraction zonisamide in serum. Side effects of anticonvulsants with large excursions of free drug in plasma or serum are known (Levy et al, 1985). Several anticonvulsants are highly protein bound and in certain situations (e.g. hypoalbuminemia) free fraction levels increase, resulting in increased side effects. Though zonisamide is not highly protein bound (40%) it is highly bound to erythrocytes (8x greater in RBCs than plasma). Furthermore DeSimone and colleagues (2005) showed that zonisamide binds to carbonic anhydrase with strong affinity but with a very slow binding rate.

This invention is not bound by theory of operation, but it is believed that the free serum zonisamide levels during the period prior to full erythrocyte binding are related to increased zonisamide side effects. This phenomenon could be considered similar to the situation when deranged serum albumin levels results in free fraction of divalproex which lead to increased adverse effects. A twice-daily sustained-release to once-weekly regimen with fewer adverse events may thus enhance compliance and prevent discontinuation of treatment. Thus, preferred embodiments are believed to satisfy the existing and long-felt need for a sustained-release formulation of zonisamide to better control the Cₘₐₓ, free serum zonisamide levels and bioavailability of the drug to reduce the instances of adverse events in patients.

### EXAMPLES

The examples below are non-limiting and are merely representative of various embodiments of the present disclosure.

### Example 1: Formulation of SR (Sustained-Release) Zonisamide

The following formulation method is an example of the preparation of a slow-release zonisamide formulation. The sustained-release formulation dissolves more slowly than, for example, the immediate-release formulation as shown in Example 3, below. Wet granulation, extrusion, spheronization and fluid-bed drying processes were utilized to produce sustained-release zonisamide pellets.

To prepare the wet granules, zonisamide HCl, microcrystalline cellulose (Avicel PH102) and methylcellulose (Methocel A15 LV), at the various percentages noted in Table 1 below, were placed into a high-shear granulator and mixed for 15 minutes. Deionized (DI) water (approx. 40 - 100 g/min) was added slowly, and the wet granules were mixed for another 5-10 minutes.

A Niro-Fielder E-140 Extruder and Niro-Fielder S-450 Spheronizer were then used to transform the wet granules into spheronized particles as follows. Three to four kilograms of wet granules were placed in a Niro-Fielder E-140 Extruder apparatus. The feeder and impeller speeds were set at 45 rpm. The extruded "spaghetti" obtained from the extruder was charged into the spheronizer having a rotation setting of 800 rpm. After 5-10 minutes of spheronization, the bead-like pellets were discharged from the spheronizer.

The spheronized pellets were then dried using a Glatt Fluid-Bed GPCG-3 dryer. The fluid bed dryer was warmed up until the product temperature reached 45°C for 5 minutes. The dryer inlet temperature that was set at between 45°C - 50°C, and the wet pellets were charged into the dryer. The drying continued until the LOD reached below 1.5%. The dried pellets were then discharged from the fluid-bed dryer and sized by passing through different screens.

The dried pellets were then encapsulated into hard gelatin capsules.

**Table 1**

| Formulation | | | |
|---|---|---|---|
| **FORMULA SUMMARY AND FINAL COMPOSITION** | | | |
| **Raw Material** | **% Formula** | | |
| | **OSF-006A** | **OSF-078 LA **** | **OSF-078 SM**** |
| Zonisamide HCl, USP | 75 | 60 | 60 |
| MicroCrystalline Cellulose (MCC Avicel PH102), NF | 20 | 35 | 35 |
| MethylCellulose (Methocel MC A15 LV Premium), USP | 5 | 5 | 5 |
| *DI Water* * | *36* | *50* | *50* |

| | | | |
|---|---|---|---|
| *Notes:* * *DI Water applied during processing is evaporated during the drying process.* ** OSF-078LA had size ranged 710- 1000 µm (#18-25 mesh); OSF-078SM had size ranged 590-710 µm (#25-30 mesh). | | | |

### Example 2: Measurements of Dissolution Rates of Various Sustained-Release Zonisamide Formulations

The percentage of dissolution of the various formulations of zonisamide were measured at various time points. The compositions were dissolved in various solutions as listed below, with a mixing rate set at 75 rpm. Note that the term "innovator" refers to commercially-available zonisamide sold under the trademark ZONEGRAN.

The OSF-006A pellets were prepared with only MCC and MC. Dissolution media contained Tween 20 and SDS in order to increase the dissolution rate. Without any SR coating, the pellets dissolved slowly. This slow dissolution property was presumaBly due to the low intrinsic solubility of zonisamide (75% conc.) in a matrix system.

The OSF-078 pellets were prepared with MCC and MC but with less zonisamide. Water was used for dissolution testing. The tested batch of OSF-078 was divided into different sizes, OSF-078 LA ~ #18 - #25 mesh and OSF-078 SM ~ #25 - #30 mesh, to investigate the effect of surface area and particle size on dissolution. The smaller sized OSF-078 SM pellets dissolved more quickly than the larger OSF-078 LA pellets. This is thought to be due the larger relative surface area of the smaller sized pellets.

Although the OSF-006A formulations were dissolved in water with Tween 20 or SDS, the dissolution rate of the OSF-078 formulation (dissolved in water only) was faster than that of OSF-006A formulation. This may be due to the lower drug concentration of the OSF-078 formulation.

**Table 2**

| Dissolution Results | | | | | | | | | |
|---|---|---|---|---|---|---|---|---|---|
| | **% Dissolved at Different Time (hrs.) @ 75 rpm** | | | | | | | | |
| Dissolution Medium | 0.25 | 0.5 | 0.75 | 1 | 2 | 4 | 6 | 8 | 10 |
| Innovator in Water | 86 | 91 | 93 | 96 | 102 | | | | |
| OSF-006A 0.5%Tween 20 | 6.2 | 10.6 | 14.2 | 17.5 | 27.6 | 41.9 | 52.5 | 60.9 | 67.7 |
| OSF-006A 2%Tween 20 | 6.8 | 11.5 | 15.3 | 18.6 | 29.2 | 44.4 | 55.5 | 64.3 | 71.3 |
| OSF-06A 0.5%SDS | 6.5 | 11.3 | 15.4 | 19.0 | 31.1 | 44.3 | 57.7 | 68.3 | 76.8 |
| OSF-006A 1%SDS | 6.8 | 11.7 | 15.8 | 19.4 | 31.9 | 45.7 | 59.5 | 71.1 | 78.6 |
| OSF-078 LA in Water | | | | 23.9 | 38.2 | 57.5 | 70.6 | | 88.2 |
| OSF-078 SM in Water | | | | 27.5 | 44.5 | 67.0 | 82.0 | | 99.2 |

### Example 3: Multiple Formulations of Zonisamide and their Dissolution Rates

To further examine the factors that influence the dissolution rate of zonisamide pellets, seventeen different formulations of zonisamide were prepared for continued testing, following the general wet granulation, extrusion, spheronization, and drying processes as described in Example 1. The formulations varied in three independent factors: zonisamide concentration, spheronizer speed, and methylcellulose concentration, as shown below in Table 3, specific size-cut pellets were encapsulated manually and subjected to dissolution rate testing.

**Table 3**

| Formulation Parameters of 17 Different Formulations of Zonisamide | | | | | | | | | | |
|---|---|---|---|---|---|---|---|---|---|---|
| | **Run #** | **API %** | **MC %** | **MCC %** | **Water %** | **API g** | **MC g** | **MCC g** | **Water g** | **Spheronizer Speed** |
| -++ | 1 | 32.20% | 4.19 | 63.61 | 72 | 322.0 | 41.9 | 636.1 | 720 | 769 |
| 000 | 2 | 50.00% | 3.00 | 47.00 | 62 | 500.0 | 30.0 | 470.0 | 620 | 650 |
| +-+ | 3 | 67.80% | 1.81 | 30.39 | 45 | 678.0 | 18.1 | 303.9 | 450 | 769 |
| 00A | 4 | 50.00% | 3.00 | 47.00 | 52 | 500.0 | 30.0 | 470.0 | 520 | 850 |
| - | 5 | 32.20% | 1.81 | 65.99 | 79 | 322.0 | 18.1 | 659.9 | 790 | 531 |
| ++- | 6 | 67.80% | 4.19 | 28.01 | 44 | 678.0 | 41.9 | 280.1 | 440 | 531 |
| a00 | 7 | 20.00% | 3.00 | 77.00 | 88 | 200.0 | 30.0 | 770.0 | 880 | 650 |
| --+ | 8 | 32.20% | 1.81 | 65.99 | 79 | 322.0 | 18.1 | 659.9 | 790 | 769 |
| 000 | 9 | 50.00% | 3.00 | 47.00 | 62 | 500.0 | 30.0 | 470.0 | 620 | 650 |
| +-- | 10 | 67.80% | 1.81 | 30.39 | 45 | 678.0 | 18.1 | 303.9 | 450 | 531 |
| 0A0 | 11 | 50.00% | 5.00 | 45.00 | 60 | 500.0 | 50.0 | 450.0 | 600 | 650 |
| 00a | 12 | 50.00% | 3.00 | 47.00 | 62 | 500.0 | 30.0 | 470.0 | 620 | 450 |
| +++ | 13 | 67.80% | 4.19 | 28.01 | 44 | 678.0 | 41.9 | 280.1 | 440 | 769 |
| 0a0 | 14 | 50.00% | 1.00 | 49.00 | 64 | 500.0 | 10.0 | 490.0 | 640 | 650 |
| A00 | 15 | 80.00% | 3.00 | 17.00 | 34 | 800.0 | 30.0 | 170.0 | 340 | 650 |
| 000 | 16 | 50.00% | 3.00 | 47.00 | 62 | 500.0 | 30.0 | 470.0 | 620 | 650 |
| -+- | 17 | 32.20% | 4.19 | 63.61 | 72 | 322.0 | 41.9 | 636.1 | 720 | 531 |

### Example 4: Measurement of Dissolution Rates of the Various Zonisamide Formulations of Table 3

Formulations prepared according to Table 3 above, were treated to the general wet granulation, extrusion, spheronization, and drying processes as described briefly in Example 1. The resulting zonisamide pellets were then encapsulated manually. The rate of dissolution was then measured at 2 hours, 4 hours, and 6 hours. Three dissolution samples were measured for each formulation, and the average dissolution was determined. Results are shown in Table 4 below.

**Table 4**

| Dissolution Rates of 17 Various Zonisamide Formulations at 2, 4 and 6 hours | | | | |
|---|---|---|---|---|
| **Zonisamide** | **LOT# OSF-J 0011-1-1A** | | | |
| Disso. | | | Time points | |
| Medium | sample | 2 Hour | 4 Hour | 6 Hour |
| | | | | |
| Water | 1 | 39.0 | 55.3 | 66.5 |
| Water | 2 | 37.6 | 53.8 | 65.0 |
| Water | 3 | 35.5 | 52.0 | 63.7 |
| | **Average** | **37.4** | **53.7** | **65.1** |
| | | | | |

| **Zonisamide** | **LOT# OSF-J 0011-2-2A** | | | |
|---|---|---|---|---|
| Water | 4 | 35.1 | 51.6 | 63.5 |
| Water | 5 | 37.7 | 54.5 | 66.5 |
| Water | 6 | 36.8 | 53.6 | 65.7 |
| | **Average** | **36.5** | **53.2** | **65.2** |
| | | | | |

| **Zonisamide** | **LOT# OSF-J0011-3-3A** | | | |
|---|---|---|---|---|
| Disso. | | | Time points | |
| Medium | sample | 2 Hour | 4 Hour | 6 Hour |
| | | | | |
| Water | 1 | 38.1 | 57.1 | 70.5 |
| Water | 2 | 37.2 | 56.1 | 69.4 |
| Water | 3 | 38.0 | 56.9 | 70.2 |
| | **Average** | **37.8** | **56.7** | **70.0** |
| | | | | |

| **Zonisamide** | **LOT# OSF-J0011-4-4A** | | | |
|---|---|---|---|---|
| Water | 4 | 37.4 | 54.6 | 66.1 |
| Water | 5 | 37.8 | 55.1 | 67.0 |
| Water | 6 | 37.8 | 55.2 | 67.5 |
| | | **Average 37.7** | **55.0** | **66.9** |
| | | | | |

| **Zonisamide** | **LOT# OSF-J0011-5-5A** | | | |
|---|---|---|---|---|
| Disso. | | | Time points | |
| Medium | sample | 2 Hour | 4 Hour | 6 Hour |
| | | | | |
| Water | 1 | 37.1 | 53.2 | 64.6 |
| water | 2 | 37.1 | 53.1 | 64.3 |
| water | 3 | 38.5 | 54.7 | 66 |
| | **Average** | **37.6** | **53.7** | **65.0** |

| **Zonisamide** | **LOT# OSF-J0011-6-6A** | | | |
|---|---|---|---|---|
| water | 4 | 31.7 | 48.9 | 61 |
| water | 5 | 32.1 | 49.7 | 61.6 |
| water | 6 | 32.1 | 49.5 | 61,4 |
| **Average** | | **32.0** | **49.4** | **61.3** |
| | | | | |

| **Zonisamide** | **LOT# OSF-J0011-7-7A** | | | |
|---|---|---|---|---|
| Disso. | | | Time points | |
| Medium | sample | 2 Hour | 4 Hour | 6 Hour |
| | | | | |
| water | 1 | 43.3 | 61.3 | 73.8 |
| water | 2 | 43.7 | 61.4 | 73.5 |
| water | 3 | 43.1 | 61.1 | 73.4 |
| | **Average** | **43.4** | **61.3** | **73.6** |
| | | | | |

| **Zonisamide** | **LOT# OSF-J0011-8A** | | | |
|---|---|---|---|---|
| water | 4 | 36.1 | 51.8 | 62.8 |
| water | 5 | 35.8 | 51.7 | 62.5 |
| water | 6 | 34.5 | 50.0 | 60.7 |
| | **Average** | **35.5** | **51.2** | **62.0** |
| | | | | |

| **Zonisamide** | **LOT# OSF-J0011-9A** | | | |
|---|---|---|---|---|
| Disso. | | | Time points | |
| Medium | sample | 2 Hour | 4 Hour | 6 Hour |
| | | | | |
| water | 1 | 40.9 | 58.1 | 70.4 |
| water | 2 | 40.7 | 58 | 70.3 |
| water | 3 | 38.7 | 56.1 | 68.7 |
| | **Average** | **40.1** | **57.4** | **69.8** |
| | | | | |

| **Zonisamide** | **LOT# OSF-J0011-10A** | | | |
|---|---|---|---|---|
| water | 4 | 39.1 | 57.6 | 70.7 |
| water | 5 | 38.4 | 56.2 | 69.1 |
| water | 6 | 40.1 | 58.8 | 72.1 |
| | **Average** | **39.2** | **57.5** | **70.6** |
| | | | | |

| **Zonisamide** | **LOT# OSF-J0011-11-11A** | | | |
|---|---|---|---|---|
| Disso. | | | Time points | |
| Medium | sample | 2 Hour | 4 Hour | 6 Hour |
| | | | | |
| water | 1 | 43.6 | 62.4 | 75.3 |
| water | 2 | 41.7 | 60.2 | 73.4 |
| water | 3 | 40.5 | 58.6 | 71.3 |
| | Average | 41.9 | 60.4 | 70.4 |
| | | | | |

| **Zonisamide** | **LOT# OSF-J0011-12-12A** | | | |
|---|---|---|---|---|
| water | 4 | 39.9 | 57.6 | 70.4 |
| water | 5 | 38.0 | 55.7 | 68.5 |
| water | 6 | 38.6 | 56.3 | 69.1 |
| Average | | 38.8 | 56.5 | 69.3 |
| | | | | |

| **Zonisamide** | **LOT# OSF-J0011-13-13A** | | | |
|---|---|---|---|---|
| Disso. | | | Time points | |
| Medium | sample | 2 Hour | 4 Hour | 6 Hour |
| | | | | |
| water | 1 | 37.1 | 55.2 | 67.8 |
| water | 2 | 36.5 | 54.6 | 67.4 |
| water | 3 | 55.2 | 55.2 | 68.1 |
| | **Average** | 42.9 | 55.0 | 67.8 |
| | | | | |

| **Zonisamide** | **LOT# OSF-J0011-14-14A** | | | |
|---|---|---|---|---|
| Disso. | | | Time points | |
| Medium | sample | 2 Hour | 4 Hour | 6 Hour |
| | | | | |
| water | 1 | 38.1 | 54.3 | 65.6 |
| water | 2 | 37.7 | 53.7 | 65.1 |
| water | 3 | 37.2 | 53.3 | 64.7 |
| | **Average** | 37.7 | 53.8 | 65.1 |
| | | | | |

| **Zonisamide** | **LOT# OSF-J0011-15-15A** | | | |
|---|---|---|---|---|
| water | 4 | 32.8 | 49.9 | 61.9 |
| water | 5 | 32.5 | 49.9 | 62.6 |
| water | 6 | 33.0 | 50.2 | 62.4 |
| | Average | 32.8 | 50.0 | 62.3 |
| | | | | |

| **Zonisamide** | **LOT# OSF-J0011-16-16A** | | | |
|---|---|---|---|---|
| Disso. | | | Time points | |
| Medium | sample | 2 Hour | 4 Hour | 6 Hour |
| | | | | |
| water | 1 | 31.5 | 47.7 | 59.5 |
| water | 2 | 30.4 | 46.3 | 58 |
| water | 3 | 31.8 | 47.8 | 59.6 |
| | Average | 31.2 | 47.3 | 59.0 |
| | | | | |

| **Zonisamide** | **LOT# OSF-J0011-17-17A** | | | |
|---|---|---|---|---|
| water | 4 | 40.3 | 57.0 | 68.9 |
| water | 5 | 39.3 | 55.9 | 67.6 |
| water | 6 | 38.8 | 56.6 | 67.7 |
| | **Average** | **39.5** | **56.5** | **68.1** |

### Example 5: Effect of Formulation Particle Size on Dissolution Rates

To determine the effect of the size of the formulation particles on the rate of dissolution, particle size distribution analysis was performed. The zonisamide pellets were prepared as described Example 1. The pellets were sized by passing through different sized mesh screens ("18 mesh", "20 mesh", and "25 mesh") to result in batches of pellets of different sizes. The dissolution rate at 2 hours, 4 hours, and 6 hours was then measured, using the method of Example 2. Results are shown in Table 5 below. The results show that for each of the tested formulations, the smaller particles (25 mesh) dissolve faster than the larger particles (18 mesh) or the unsized particles.

**Table 5**

| Effect of Particle Size on Dissolution Rate | | | | |
|---|---|---|---|---|
| **Zonisamide** | **LOT# OSF-J0011-2-2A** | | | |
| Disso. | | | Time points | |
| Medium | sample | 2 Hour | 4 Hour | 6 Hour |
| | | | | |
| water | 4 | 35.1 | 51.6 | 63.5 |
| water | 5 | 37.7 | 54.5 | 66.5 |
| water | 6 | 36.8 | 53.6 | 65.7 |
| | **Average** | **36.5** | **53.2** | **65.2** |
| | | | | |

| **Zonisamide** | **LOT# OSF-J0011-2-2A- (sized to 18 Mesh)** | | | |
|---|---|---|---|---|
| Disso. | | | Time points | |
| Medium | sample | 2 Hour | 4 Hour | 6 Hour |
| | | | | |
| water | 1 | 35.0 | 50.1 | 60.5 |
| water | 2 | 32.5 | 47.8 | 58.5 |
| water | 3 | 33.5 | 48.8 | 59.2 |
| | **Average** | **33.7** | **48.9** | **59.4** |
| | | | | |

| **Zonisamide** | **LOT# OSF-J0011-2-2A (sized to 25 Mesh)** | | | |
|---|---|---|---|---|
| water | 4 | 42.1 | 61.3 | 74.3 |
| water | 5 | 40.2 | 59.8 | 72.9 |
| water | 6 | 40.2 | 59.7 | 72.7 |
| | **Average** | **40.8** | **60.3** | **73.3** |
| | | | | |

| **Zonisamide** | **LOT# OSF-J0011-5-5A** | | | |
|---|---|---|---|---|
| Disso. | | | Time points | |
| Medium | sample | 2 Hour | 4 Hour | 6 Hour |
| | | | | |
| | | | | |
| water | 1 | 37.1 | 53.2 | 64.6 |
| water | 2 | 37.1 | 53.1 | 64.3 |
| water | 3 | 38.5 | 54.7 | 66 |
| | **Average** | **37.6** | **53.7** | **65.0** |

| **Zonisamide** | **LOT# OSF-J0011-5-5A-(sized to 18 Mesh)** | | | |
|---|---|---|---|---|
| Disso. | | | Time points | |
| Medium | sample | 2 Hour | 4 Hour | 6 Hour |
| | | | | |
| water | 1 | 34.7 | 49.3 | 59.4 |
| water | 2 | 35.5 | 50.0 | 60.0 |
| water | 3 | 35.1 | 49.8 | 59.9 |
| | **Average** | **35.1** | **49.7** | **59.8** |
| | | | | |

| **Zonisamide** | **LOT# OSF-J0011-5-5A-(sized to 25 Mesh)** | | | |
|---|---|---|---|---|
| water | 4 | 42.9 | 60.9 | 73.5 |
| water | 5 | 41.7 | 60.1 | 73.0 |
| water | 6 | 42.1 | 60.4 | 72.9 |
| | **Average** | **42.2** | **60.5** | **73.1** |
| | | | | |

| **Zonisamide** | **LOT# OSF-J0011-13-13A** | | | |
|---|---|---|---|---|
| Disso. | | | Time points | |
| Medium | sample | 2 Hour | 4 Hour | 6 Hour |
| | | | | |
| water | 1 | 37.1 | 55.2 | 67.8 |
| water | 2 | 36.5 | 54.6 | 67.4 |
| water | 3 | 55.2 | 55.2 | 68.1 |
| | **Average** | **42.9** | **55.0** | **67.8** |

| **Zonisamide** | **LOT# OSF-J0011-13-13A- (sized to 18 Mesh)** | | | |
|---|---|---|---|---|
| Disso. | | | Time points | |
| Medium | sample | 2 Hour | 4 Hour | 6 Hour |
| | | | | |
| water | 1 | 33.3 | 49.2 | 60.5 |
| water | 2 | 33.5 | 49.7 | 61.0 |
| water | 3 | 32.9 | 49.2 | 60.7 |
| | **Average** | **33.2** | **49.4** | **60.7** |
| | | | | |

| **Zonisamide** | **LOT# OSF-J0011-13-13A- (sized to 25 Mesh)** | | | |
|---|---|---|---|---|
| water | 4 | 47.4 | 69.0 | 83.1 |
| water | 5 | 44.1 | 65.3 | 79.2 |
| water | 6 | 43.2 | 65 | 79.6 |
| | **Average** | **44.9** | **66.4** | **80.6** |

### Example 6

**Table 6: Composition of Zonisamide SR (Sustained-Release) Tablets**

| **Component** | **Fast Release Composition (amount per tablet)** | **Medium Release Composition (amount per tablet)** | **Slow Release Composition (amount per tablet)** |
|---|---|---|---|
| Zonisamide | 120 mg | 120 mg | 120 mg |
| Microcrystalline Cellulose, NF | 130 mg | 126 mg | 120 mg |
| Lactose, Anhydrous, NF | 57 mg | 54 mg | 46 mg |
| Hydroxypropyl Cellulose, NF (Klucel HXF) | 21 mg | 28 mg | 42 mg |
| Crospovidone, NF | 14 mg | 14 mg | 14 mg |
| Magnesium Stearate, NF | 6 mg | 6 mg | 6 mg |
| Colloidal Silicon Dioxide, NF | 2 mg | 2 mg | 2 mg |
| Total Tablet Weight = | 350 mg | 30 mg | 350 mg |

Three zonisamide 120 mg sustained-release formulations were formed into tablets in the usual fashion using the ingredients shown in Table 6. Dissolution results (37° with stirring in water) are shown in Table 7 below:

**Table 7: Dissolution Rates of Sustained-Release 120 mg Tablet**

| | **Fast Release** | **Medium Release** | **Slow Release** |
|---|---|---|---|
| | **% dissolved** | **% dissolved** | **% dissolved** |
| 1 hr | 61 | 36 | 23 |
| 2 hr | 72 | 50 | 29 |
| 4 hr | 82 | 64 | 37 |
| 8 hr | 91 | 79 | 52 |
| 12 hr | | | 63 |
| 24 hr | | | |

The data in Table 7 shows that the sustained-release zonisamide formulations in the form of tablets can be prepared, and that the dissolution profiles of the tablets can be controlled by controlling the amount of retardant excipient (hydroxypropyl cellulose).

### Example 7

A single-center, double-blind, double-dummy, placebo-controlled, two-period, two-sequence crossover study of immediate-release (IR) zonisamide and three sustained-release (SR) formulations was performed on 36 healthy, normal volunteers. Subjects were randomized to receive one of the three zonisamide SR formulations (described in Table 6 above) and an IR formulation in 1:1:1 ratio. A total of 12 subjects were randomized to each group, for a total of 36 subjects. Within each formulation, subjects were randomly assigned to receive the SR and IR formulations in one of two sequences: half of the subjects (n = 6) received the SR formulation in period 1 followed by the IR formulation in period 2; the remaining subjects (n = 6) received the treatments in the reverse order. Serial blood samples were collected pre-dose and at multiple time points post-dose. Samples were analyzed by validated LC-MS/MS methods. The administration of study drug in each period was separated by a 21-day washout period. PK parameters were determined and are summarized in Table 8.

**Table 8:**

| **Formulation, dose** | **Parameter** | **Mean ± SD** **Total Serum** |
|---|---|---|
| | AUC₍₀₋ₗₐₛₜ₎ (hr·ng/mL) | 62376 ± 13087 |
| Immediate- | Cₘₐₓ(ng/mL) | 840 ±186 |
| release | tₘₐₓ(hr) | 5.2 ± 5.4 |
| 100mg | t_{½} (hr) | 71 ± 22 |
| | AUC0-∞ (hr·ng/mL) | 84020 ±2 3800 |
| | AUC₍₀₋ₗₐₛₜ₎ (hr**·**ng/mL) | 71756 ± 6785 |
| Sustained-Release | Cₘₐₓ (ng/mL) | 929 ± 247 |
| Slow | tₘₐₓ (hr) | 4.3 ± 2.9 |
| 120mg | t_{½} (hr) | 67 ± 16 |
| | AUCO-oo (hr·ng/mL) | 94014 ± 15177 |
| Sustained-Release | AUC₍₁₂ₕᵣ₎ (hr·ng/mL) | 68325 |
| Slow at Steady state | This yields 90% of AUC for IR at 100mg Steady State | |
| 90 mg | Cₘₐₓ (ng/mL) | 7957 |
| (Model Predicted) | This yields 84% of Cₘₐₓ for IR at 100 mg Steady State | |

Subjects receiving a 100 mg oral dose of the immediate-release zonisamide produced an area under the serum concentration time curve of approximately 62376 hr*ng/mL, while subjects receiving a 120 mg oral dose of the sustained-release zonisamide produced an area under the serum concentration time curve of approximately 71756 hr*ng/mL. The maximum observed serum concentrations were 840 and 929 ng/mL for the immediate-release dosage forms and sustained-release dosage forms, respectively. When the data are utilized to model pharmacokinetic behavior of the immediate and the sustained-release dosage forms at steady state, the predicted area under the serum concentration time curve for the sustained-release dose form at 90 mg is 90% of the similar curve for the immediate-release form. In a similar manner, the maximum predicted serum concentrations for the sustained-release dose form at 90 mg is 84% of the immediate-release form.

Sustained-release zonisamide dosage forms having very different in vitro release profiles, as characterized by standard parameters such as percentage released in 1, 4, 8 and 12 hours, can have in vivo PK profiles that are similar to immediate-release dosage forma, but have in vivo PK profiles that differ in very meaningful ways at steady state. Specifically, there is greater bioavailabilty as defined by Area under the serum concentration curve at steady state and lower Cₘₐₓ at steady state than a conventional immediate-release formulation at the same dose.

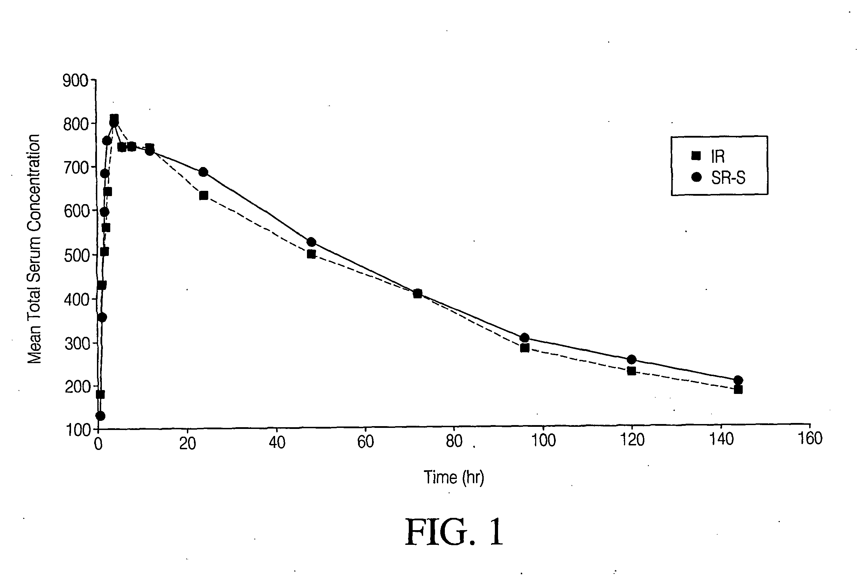

Figure 1 illustrates dose-normalized total serum concentration time profiles for zonisamide immediate-release (IR) and zonisamide sustained-release slow (SR-S) formulations following a single oral dose as a function of time. The mean total serum concentrations are plotted over time. The illustrated data represent the mean total serum concentration values for each treatment group among the subjects described above.

Various measures of steady state PK parameters for the SR formulation as compared to the IR formulations are shown in Tables 9-12. For example, Table 9 shows the percentage of Cₘₐₓ obtained for some of the sustained-release formulations as compared to the immediate-release formulations.

**Table 9: Cₘₐₓ (steady state)**

| | **SR = 90 mg** | **SR = 100 mg** |
|---|---|---|
| | **IR = 100 mg** | **IR = 100 mg** |
| SR-Slow | 84% | 93% |
| SR-Fast | 66% | 73% |
| SR-Medium | 64% | 71% |

**Table 10: AUC(0-24) (steady state)**

| | **SR = 90mg** | **SR = 100 mg** |
|---|---|---|
| | **IR = 100mg** | **IR = 100mg** |
| SR-Slow: | 90% | 100% |
| SR-Fast: | 75% | 83% |
| SR-Medium: | 77% | 86% |

**Table 11: Cₘₐₓ (single dose)**

| | **SR = 120mg** | **SR = 100 mg** |
|---|---|---|
| | **IR = 100mg** | **IR = 100mg** |
| SR-Slow: | 122% | 102% |
| SR-Fast: | 113% | 94% |
| SR-Medium: | 115% | 96% |

**Table 12: AUC₀₋₂₄ (single dose)**

| | **SR = 120mg** | **SR = 100 mg** |
|---|---|---|
| | **IR = 100mg** | **IR = 100mg** |
| SR-Slow: | 112% | 93% |
| SR-Fast: | 113% | 94% |
| SR-Medium: | 114% | 95% |

In the study adverse events were reported by 44% of the immediate-release subjects versus only 8% of the SR-S group. The most pronounced difference in adverse event reporting as a function of treatment group was a substantial decrease in frequency of headaches observed; subjects receiving immediate-release zonisamide reported 7 headaches whereas no headaches were reported in the SR-S group.

For all subjects, the most common adverse events included: Headache (22%), sleepiness (17%), tiredness/fatigue (11%) and frequent urination (6%). Single episodes of the following events were also reported: vomiting, leg cramps, increased dream activity, diarrhea, dry mouth, nausea, muscle tension, left hip/leg pain, polyuria, headache over the left eye, and dizziness. Two episodes of difficulty reaching erection and premature ejaculation were reported in the same subject. For the SR-S group, only 3 adverse events were reported; these were single episodes of increased dream activity, diarrhea and sleepiness. No serious adverse events were reported; one subject withdrew consent after the first treatment period due to extenuating family circumstances.

Because the frequency of spontaneously reported headaches was dramatically decreased between subjects receiving the immediate-release formulation (44%) and the sustained-release slow formulation (8%), the pharmacokinetics of zonisamide for these subjects was examined in more detail. Specifically, the subjects reporting headache in the immediate-release treatment group were compared with the sustained-release slow subjects who did not report an incidences of headache.

When the total serum concentration data are corrected for differences in dose between the immediate-release formulation and the sustained-release slow formulation, there are several observed trends. First, there is a tendency toward higher exposure in subjects receiving immediate-release that reported headache (933 ng/mL), relative to those subjects receiving immediate-release that did not report headache (768 ng/mL). This trend continues for the area under the serum concentration time curve (68629 ng*hr/mL vs 61237 ng*hr/mL, respectively). Second, for subjects receiving the sustained-release formulation there is a decreased exposure in terms of Cₘₐₓ and AUC, relative to subjects that reported headache (800 ng/mL vs 933 ng/mL); this exposure was similar to subjects receiving immediate-release formulation that did not report headache (800 ng/mL vs 768 ng/mL).

When the whole blood zonisamide concentration data are corrected for differences in dose between the immediate-release formulation and the sustained-release slow formulation, there are several observed trends. Unlike total serum, there is no tendency toward higher exposure in subjects receiving immediate-release that reported headache (Cₘₐₓ 7167 ng/mL; AUCall 636853 ng*hr/mL)), relative to those subjects receiving immediate-release that did not report headache (Cₘₐₓ 6915 ng/mL; AUCall 588577 ng*hr/mL). There is, however, a decrease in exposure for subjects receiving the sustained-release slow formulation relative to immediate-release formulation (Cₘₐₓ 6381 ng/mL; AUCall 571326 ng*hr/mL).

The differences in exposure are particularly marked when the free serum zonisamide fraction is assessed (Cₘₐₓ 718 ng/mL; AUCall 55467 ng*hr/mL). This reduction in the free serum fraction is believed to be correlated to the reduction in side effects observed with the zonisamide SR-slow formulation. See Table 13 below.

**Table 13**

| | **Total ZNS** | | **Whole Blood ZNS** | | **Free ZNS** | |
|---|---|---|---|---|---|---|
| | **Cₘₐₓ** | **AUCₐₗₗ** | **Cₘₐₓ** | **AUCₐₗₗ** | **Cₘₐₓ** | **AUCₐₗₗ** |
| | **(ng/mL)** | **(ng-hr/mL)** | **(ng/mL)** | **(ng-hr/mL)** | **(ng/mL)** | **(ng-hr/mL)** |
| IR - Headache | 933 | 68629 | 6915 | 588577 | 858 | 631773 |
| IR - No Headache | 768 | 61237 | 7167 | 636853 | 714 | 57757 |
| SR-S - No Headache | 800 | 59895 | 6381 | 571326 | 718 | 55467 |

| **ZB 222 Adverse Event Table (subject numbers)** | | | | | | |
|---|---|---|---|---|---|---|
| **Reported Event** | **Immediate-release** | | **SR - Slow** | | **SR - Med** | **SR - Fast** |
| Headache | 1,7,15,17,23,26 | | 0 | | 23 | 0 |
| Headache over L eye | 0 | | 0 | | 29 | 0 |

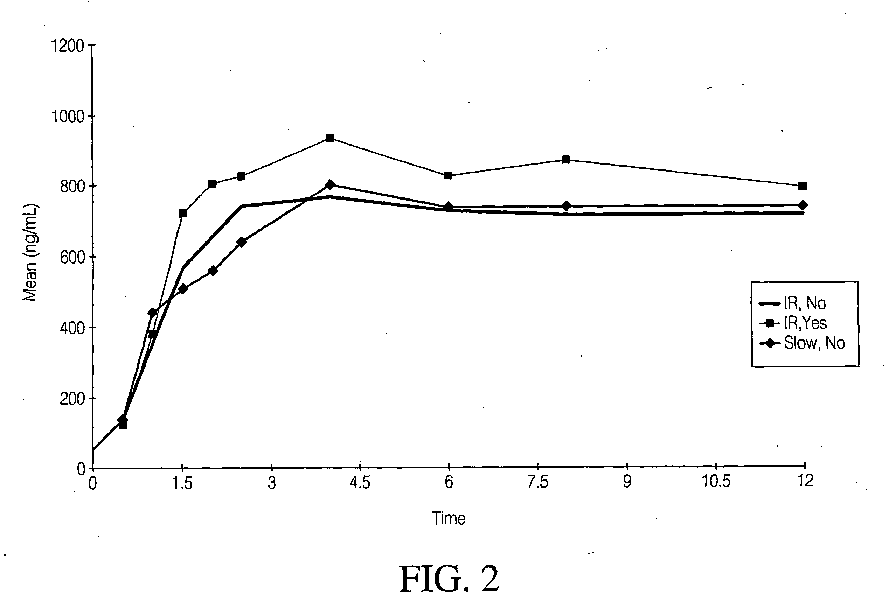

Figure 2 illustrates Cₘₐₓ of total serum zonisamide as a function of time for the subject groups reported in Table 13. "IR, No" represents the total serum zonisamide for subjects taking immediate-release zonisamide who reported no headaches. "IR, Yes" represents the total serum zonisamide for subjects taking immediate-release zonisamide who reported headaches. "Slow, No" represents the total serum zonisamide for subjects who were taking a sustained-release zonisamide who spontaneously reported headaches.

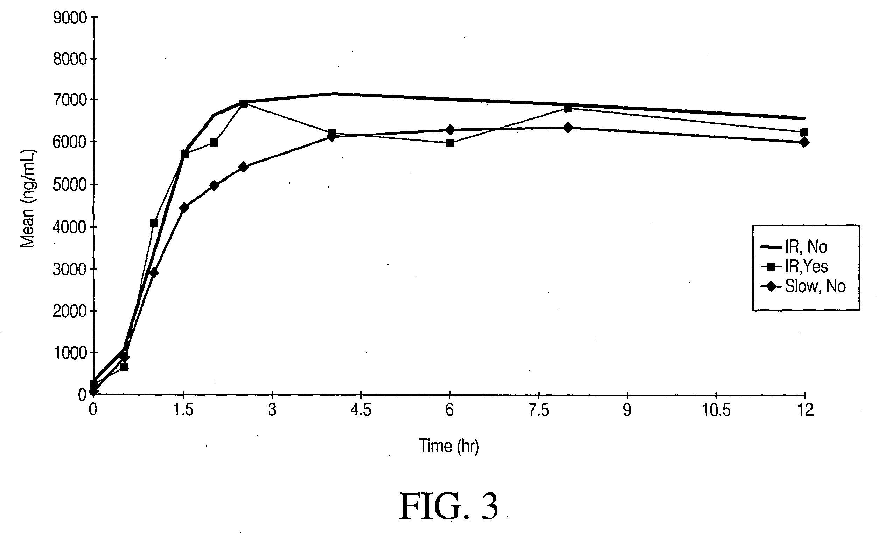

Figure 3 illustrates Cₘₐₓ of whole blood zonisamide as a function of time for the subject groups reported in Table 13. "IR, No" represents the whole blood zonisamide for subjects taking immediate-release zonisamide who reported no headaches. "IR, Yes" represents the whole blood zonisamide for subjects taking immediate-release zonisamide who reported headaches. "Slow, No" represents the whole blood zonisamide for subjects who were taking a sustained-release zonisamide who spontaneously reported headaches.

Those skilled in the art, informed by the guidance provided herein, can prepare sustained-release zonisamide formulations having a wide range of dissolution and pharmacokinetic parameters.

Sustained-release zonisamide pharmaceutical formulation can be used to treat various conditions. For example, an embodiment provides a method for affecting weight loss, increasing energy expenditure, increasing satiety in an individual, and/or suppressing the appetite of an individual, comprising identifying an individual in need thereof and administering effective amounts of sustained-release zonisamide, e.g., by administering any of the sustained-release zonisamide pharmaceutical formulations described herein, by any one or more of the various routes of administration described herein.

### Combinations Comprising Sustained-Release Zonisamide and Bupropion

An embodiment provides a pharmaceutical formulation comprising sustained-release zonisamide and bupropion, e.g., sustained-release bupropion. Bupropion, whose chemical name is (±)-1-(3-chlorophenyl)-2-[(1,1-dimethylethyl)amino]-1-propanone, is the active ingredient in the drugs marketed as ZYBAN^{®} and WELLBUTRIN^{®}, and is usually administered as a hydrochloride salt. Throughout the present disclosure, whenever the term "bupropion" is used, it is understood that the term encompasses bupropion as a free base, or as a physiologically acceptable salt thereof, or as a bupropion metabolite or salt thereof.

The metabolites of bupropion suitable for inclusion in the methods and compositions described herein include the erythro- and threo-amino alcohols of bupropion, the erythro-amino diol of bupropion, and morpholinol metabolites of bupropion. In some embodiments, the metabolite of bupropion is (±)-(2R*,3R*)-2-(3-chlorophenyl)-3,5,5-trimethyl-2-morpholinol. In some embodiments the metabolite is (-)-(2R*,3R*)-2-(3-chlorophenyl)-3,5,5-trimethyl-2-morpliolinol, while in other embodiments, the metabolite is (+)-(2S,3S)-2-(3-chlorophenyl)-3,5,5-trimethyl-2-morpholinol. Preferably, the metabolite of bupropion is (+)-(2S,3S)-2-(3-chlorophenyl)-3,5,5-trimethyl-2-morpholinol, which is known by its common name of radafaxine. The scope of the present disclosure includes the above-mentioned metabolites of bupropion as a free base or as a physiologically acceptable salt thereof. Sustained-release bupropion formulations of bupropion are known in the art. See, for example, U.S. Patent 6,905,708, which discloses a once-daily dosage configured to deliver bupropion in vivo over a 6 to 12 hour period.

A pharmaceutical formulation comprising sustained-release zonisamide and bupropion can be made in various ways, e.g., by intermixing granules or beads of sustained-release zonisamide with bupropion or sustained-release bupropion, then forming tablets from the mixture in the usual fashion.

Sustained-release zonisamide pharmaceutical formulation can be used in combination with bupropion to treat various conditions. For example, an embodiment provides a method for affecting weight loss, increasing energy expenditure, increasing satiety in an individual, and/or suppressing the appetite of an individual, comprising identifying an individual in need thereof and administering effective amounts of sustained-release zonisamide and bupropion. In some embodiments the sustained-release zonisamide and bupropion are administered more or less simultaneously. In other embodiments the sustained-release zonisamide is administered prior to the bupropion. In yet other embodiments, the sustained-release zonisamide is administered subsequent to the bupropion. In other embodiments, one of the compounds is administered while the other compound is being administered.

### Combinations Comprising Sustained-Release Zonisamide and Naltrexone

An embodiment provides a pharmaceutical formulation comprising sustained-release zonisamide and naltrexone, e.g., sustained-release naltrexone. Naltrexone (17-(cyclopropylmethly)-4, 5α-epoxy- 3, 14-dihydroxymorphinan-6-one) is an opioid receptor antagonist used primarily in the management of alcohol dependence and opioid dependence. Mu-subtype selective opioid antagonists such as naltrexone are also of considerable current interest as agents for the treatment of obesity (Glass, M. J.; Billington, C. J.; Levine, A. S. Neuropeptides 1999, 33, 350) and CNS disorders (Reneric, J. P.; Bouvard, M. P. CNS Drugs 1998, 10, 365).

Naltrexone is marketed as its hydrochloride salt, naltrexone hydrochloride, under the trade name REVIA™. REVIA™ is an immediate-release formulation of naltrexone, with 50 mg strength. The maximum serum concentration of immediate-release naltrexone is reached very rapidly, typically a Tₘₐₓ of approximately 1 hour. Immediate-release naltrexone can induce side effects such as nausea, which is attributable to the maximum blood plasma concentration levels (Cₘₐₓ).

Formulations of sustained-release naltrexone have been disclosed in U.S. Provisional Patent Application Serial No. 60/811,251, filed June 5, 2006, which is hereby incorporated by reference in its entirety. In some embodiments, oral dosage forms of naltrexone are effective to provide an AUC between about 75% to 125% of 50 mg immediate-release naltrexone tablets. In some embodiments oral dosage forms of naltrexone provide an amount of a retardant excipient that is effective to provide a Cₘₐₓ that is less than or equal to about 80% of the Cₘₐₓ of 50 mg immediate-release naltrexone tablets.

Those skilled in the art informed by the guidance provided herein can formulate oral dosage forms described herein. For example, one skilled in the art could formulate an oral dosage form that comprises an amount of naltrexone that is effective to provide an AUC between about 75-125% of 50 mg immediate-release naltrexone tablets, and an amount of an appropriate retardant excipient effective to provide a Cₘₐₓ that is less than or equal to about 80% of the Cₘₐₓ of 50 mg immediate-release naltrexone tablets. Further, given the guidance provided herein, the skilled artisan could formulate an oral dosage form having a pharmacodynamic profile characterized by occupation of greater than or equal to 80% of the opioid receptors in the brain as measured by positron emission tomography (PET).

A pharmaceutical formulation comprising sustained-release zonisamide and naltrexone can be made in various ways, e.g., by intermixing granules or beads of sustained-release zonisamide with naltrexone or sustained-release naltrexone, then forming tablets from the mixture in the usual fashion.

Sustained-release zonisamide pharmaceutical formulation can be used in combination with naltrexone to treat various conditions. For example, an embodiment provides a method for affecting weight loss, increasing energy expenditure, increasing satiety in an individual, and/or suppressing the appetite of an individual, comprising identifying an individual in need thereof and administering effective amounts of sustained-release zonisamide and naltrexone. In some embodiments the sustained-release zonisamide and naltrexone are administered more or less simultaneously. In other embodiments the sustained-release zonisamide is administered prior to the naltrexone. In yet other embodiments, the sustained-release zonisamide is administered subsequent to the naltrexone. In other embodiments, one of the compounds is administered while the other compound is being administered.

## Claims

1. A sustained-release pharmaceutical formulation comprising:
zonisamide; and
a retardant excipient, wherein the pharmaceutical formulation has a sustained-release dissolution profile comprising at least one dissolution characteristic selected from:
(a) less than 70% of the zonisamide in the sustained-release pharmaceutical formulation is dissolved within a first hour in a standard dissolution test, and
(b) less than 75% of the zonisamide in the sustained-release pharmaceutical formulation is dissolved within a second hour in a standard dissolution test,
wherein said pharmaceutical formulation comprises at least 5% by weight of said retardant excipient.

2. The sustained-release pharmaceutical formulation of Claim 1, wherein the dissolution characteristic is less than 40% of the zonisamide in the sustained-release pharmaceutical formulation is dissolved within a first hour in a standard dissolution test.

3. The sustained-release pharmaceutical formulation of Claim 1, wherein the dissolution characteristic is less than 30% of the zonisamide in the sustained-release pharmaceutical formulation is dissolved within a first hour in a standard dissolution test.

4. The sustained-release pharmaceutical formulation of Claim 1, wherein the dissolution characteristic is less than 55% of the zonisamide in the sustained-release pharmaceutical formulation is dissolved within a second hour in a standard dissolution test.

5. The sustained-release pharmaceutical formulation of Claim 1, wherein the dissolution characteristic is less than 35% of the zonisamide in the sustained-release pharmaceutical formulation is dissolved within a second hour in a standard dissolution test.

6. The pharmaceutical formulation of any of Claims 1 to 5, wherein said sustained-release zonisamide pharmaceutical formulation is in the form of beads, and wherein the beads further comprise a filler.

7. The pharmaceutical formulation of Claim 6, wherein said dissolution characteristic is less than 65% of the zonisamide in the sustained-release zonisamide beads is dissolved within the first hour in a standard dissolution test.

8. The pharmaceutical formulation of Claim 6, wherein said dissolution characteristic is less than 50% of the zonisamide in the sustained-release zonisamide beads is dissolved within the first hour in a standard dissolution test.

9. The pharmaceutical formulation of any of Claims 1 to 8, wherein said retardant excipient comprises at least one selected from the group consisting of hydroxypropyl methylcellulose (HPMC), hydroxyethylcellulose, hydroxypropylcellulose (HPC), methylcellulose, ethylcellulose, cellulose acetate butyrate, cellulose acetate phthalate, hydroxypropylmethyl cellulose phthalate, microcrystalline cellulose, corn starch, polyethylene oxide, polyvinyl alcohol (PVA), polyvinylpyrrolidone (PVP), cross-linked PVP, polyvinyl acetate phthalate, polyethylene glycol, zein, poly-DL-lactide-co-glycolide, dicalcium phosphate, calcium sulfate, and mixtures thereof.

10. The pharmaceutical formulation of any one of Claims 1 to 9, wherein said pharmaceutical formulation comprises at least 10% by weight of said retardant excipient.

11. The pharmaceutical formulation of any one of Claims 1 to 10, wherein said retardant excipient is configured to provide, upon administration to a patient, an average free serum zonisamide Cₘₐₓ value that is less than an average free serum zonisamide Cₘₐₓ value of a comparable immediate-release zonisamide under comparable conditions.

12. The pharmaceutical formulation of any one of Claims 1 to 10, wherein said retardant excipient is configured to provide, upon administration to a patient, an average free serum zonisamide Cₘₐₓ value is at least 5% less than an average free serum zonisamide Cₘₐₓ value of a comparable immediate-release zonisamide under comparable conditions.

13. The pharmaceutical formulation of any one of Claims 1 to 12, further comprising bupropion.

14. The pharmaceutical formulation of any one of Claims 1 to 13, configured in a dosage form selected from twice daily, once daily, once every two days, once every three days, once every four days, once every five days, once every six days, and once weekly.

15. Use of a pharmaceutical formulation of any one of Claims 1 to 14 in the preparation of a medicament for affecting weight loss, increasing energy expenditure, increasing satiety or suppressing appetite.

16. Use of a pharmaceutical formulation of any one of Claims 1 to 14 in the preparation of a medicament for reducing risk of an adverse event associated with administering a comparable dosage of an immediate-release zonisamide.

## Patentansprüche

1. Pharmazeutische Formulierung mit verzögerter Freisetzung, umfassend:
Zonisamid; und
einen verzögernden Exzipienten, wobei die pharmazeutische Formulierung ein Auflösungsprofil mit verzögerter Freisetzung aufweist, das mindestens ein Auflösungsmerkmal umfasst, das aus den folgenden ausgewählt ist:
(a) weniger als 70% des Zonisamids in der pharmazeutischen Formulierung mit verzögerter Freisetzung werden bei einem Standard-Auflösungstest innerhalb einer ersten Stunde gelöst, und
(b) weniger als 75% des Zonisamids in der pharmazeutischen Formulierung mit verzögerter Freisetzung werden bei einem Standard-Auflösungstest innerhalb einer zweiten Stunde gelöst,
wobei die pharmazeutische Formulierung mindestens 5 Gew.-% des genannten verzögernden Exzipienten enthält.

2. Die Pharmazeutische Formulierung mit verzögerter Freisetzung nach Anspruch 1, wobei das Auflösungsmerkmal darin besteht, dass weniger als 40% des Zonisamids in der pharmazeutischen Formulierung mit verzögerter Freisetzung bei einem Standard-Auflösungstest innerhalb einer ersten Stunde gelöst werden.

3. Die Phärmazeutische Formulierung mit verzögerter Freisetzung nach Anspruch 1, wobei das Auflösungsmerkmal darin besteht, dass weniger als 30% des Zonisamids in der pharmazeutischen Formulierung mit verzögerter Freisetzung bei einem Standard-Auflösungstest innerhalb einer ersten Stunde gelöst werden.

4. Die Pharmazeutische Formulierung mit verzögerter Freisetzung nach Anspruch 1, wobei das Auflösungsmerkmal darin besteht, dass weniger als 55% des Zonisamids in der pharmazeutischen Formulierung mit verzögerter Freisetzung bei einem Standard-Auflösungstest innerhalb einer zweiten Stunde gelöst werden.

5. Die Pharmazeutische Formulierung mit verzögerter Freisetzung nach Anspruch 1, wobei das Auflösungsmerkmal darin besteht, dass weniger als 35% des Zonisamids in der pharmazeutischen Formulierung mit verzögerter Freisetzung bei einem Standard-Auflösungstest innerhalb einer zweiten Stunde gelöst werden.

6. Die Pharmazeutische Formulierung nach einem der Ansprüche 1 bis 5, wobei die pharmazeutische Zonisamid-Formulierung mit verzögerter Freisetzung in Form von Kügelchen vorliegt und wobei die Kügelchen ferner einen Füllstoff umfassen.

7. Die Pharmazeutische Formulierung nach Anspruch 6, wobei das Auflösungsmerkmal darin besteht, dass weniger als 65% des Zonisamids in den Zonisamid-Kügelchen mit verzögerter Freisetzung bei einem Standard-Auflösungstest innerhalb der ersten Stunde gelöst werden.

8. Die Pharmazeutische Formulierung nach Anspruch 6, wobei das Auflösungsmerkmal darin besteht, dass weniger als 50% des Zonisamids in den Zonisamid-Kügelchen mit verzögerter Freisetzung bei einem Standard-Auffösungstest innerhalb der ersten Stunde gelöst werden.

9. Die Pharmazeutische Formulierung nach einem der Ansprüche 1 bis 8, wobei der verzögernde Exzipient mindestens einen Stoff umfasst, der aus der Gruppe ausgewählt ist, die aus Folgendem besteht:
Hydroxypropylmethylcellulose (HPMC), Hydroxyethylcellulose, Hydroxypropylcellulose (HPC), Methylcellulose, Ethylcellulose, Cellulosacetatbutyrat, Celluloseacetatphthalat, Hydroxypropylmethylcellulosephthalat, mikrokristalline Cellulose, Maisstärke, Polyethylenoxid, Polyvinylalkohol (PVA), Polyvinylpyrrolidon (PVP), vernetztes PVP, Polyvinylacetatphthalat, Polyethylenglycol, Zein, Poly-DL-lactid-co-glycolid, Dicalciumphosphat, Calciumsulfat und Mischungen davon.

10. Die Pharmazeutische Formulierung nach einem der Ansprüche 1 bis 9, wobei die pharmazeutische Formulierung mindestens 10 Gew.-% des genannten verzögernden Exzipienten enthält.

11. Die Pharmazeutische Formulierung nach einem der Ansprüche 1 bis 10, wobei der verzögernde Exzipient derart aufgebaut ist, dass er bei Verabreichung an einen Patienten einen mittleren Cₘₐₓ-Wert an freiem Zonisamid im Serum hervorruft, der niedriger ist als ein mittlerer Cₘₐₓ-Wert an freiem Zonisamid im Serum, der mit einem vergleichbaren Zonisamid mit sofortiger Freisetzung unter vergleichbaren Bedingungen erzielt wird.

12. Die Pharmazeutische Formulierung nach einem der Ansprüche 1 bis 10, wobei der verzögernde Exzipient derart aufgebaut ist, dass er bei Verabreichung an einen Patienten einen mittleren Cₘₐₓ-Wert an freiem Zonisamid im Serum hervorruft, der um mindestens 5% niedriger ist als ein mittlerer Cₘₐₓ-Wert an freiem Zonisamid im Serum, der mit einem vergleichbaren Zonisamid mit sofortiger Freisetzung unter vergleichbaren Bedingungen erzielt wird.

13. Die Pharmazeutische Formulierung nach einem der Ansprüche 1 bis 12, ferner umfassend Bupropion.

14. Die Pharmazeutische Formulierung nach einem der Ansprüche 1 bis 13, die in einer Dosierungsform vorliegt, die aus Folgendem ausgewählt ist: zwei Mal pro Tag, ein Mal pro Tag, ein Mal alle zwei Tage, ein Mal alle drei Tage, ein Mal alle vier Tage, ein Mal alle fünf Tage, ein Mal alle sechs Tage und ein Mal pro Woche.

15. Verwendung einer pharmazeutischen Formulierung nach einem der Ansprüche 1 bis 14 zur Herstellung eines Arzneimittels zur Beeinflussung des Gewichtsverlustes, zur Erhöhung des Energieverbrauchs, zur Verstärkung des Sättigungsgefühls oder zur Unterdrückung des Appetits.

16. Verwendung einer pharmazeutischen Formulierung nach einem der Ansprüche 1 bis 14 zur Herstellung eines Arzneimittels zur Senkung des Risikos des Auftretens einer Nebenwirkung, die mit der Verabreichung einer vergleichbaren Dosis von Zonisamid mit sofortiger Freisetzung verbunden ist.

## Revendications

1. Formulation pharmaceutique à libération prolongée comprenant :
du zonisamide ; et
un excipient retard, dans lequel la formulation pharmaceutique a un profil de dissolution à libération prolongée comprenant au moins une caractéristique de dissolution choisie parmi :
(a) moins de 70% du zonisamide dans la formulation pharmaceutique à libération prolongée sont dissous dans la première heure lors d'un essai de dissolution type, et
(b) moins de 75% du zonisamide dans la formulation pharmaceutique à libération prolongée sont dissous dans la deuxième heure lors d'un essai de dissolution type,
dans laquelle ladite formulation pharmaceutique comprend au moins 5% en masse dudit excipient retard.

2. Formulation pharmaceutique à libération prolongée selon la revendication 1, dans laquelle la caractéristique de dissolution est que moins de 40% du zonisamide dans la formulation pharmaceutique à libération prolongée sont dissous dans la première heure lors d'un essai de dissolution type.

3. Formulation pharmaceutique à libération prolongée selon la revendication 1, dans laquelle la caractéristique de dissolution est que moins de 30% du zonisamide dans la formulation pharmaceutique à libération prolongée sont dissous dans la première heure lors d'un essai de dissolution type.

4. Formulation pharmaceutique à libération prolongée selon la revendication 1, dans laquelle la caractéristique de dissolution est que moins de 55% du zonisamide dans la formulation pharmaceutique à libération prolongée sont dissous dans la deuxième heure lors d'un essai de dissolution type.

5. Formulation pharmaceutique à libération prolongée selon la revendication 1, dans laquelle la caractéristique de dissolution est que moins de 35% du zonisamide dans la formulation pharmaceutique à libération prolongée sont dissous dans la deuxième heure lors d'un essai de dissolution type.

6. Formulation pharmaceutique selon l'une quelconque des revendications 1 à 5, dans laquelle ladite formulation pharmaceutique de zonisamide à libération prolongée se présente sous la forme de billes, et dans laquelle les billes comprennent en outre une charge.

7. Formulation pharmaceutique selon la revendication 6, dans laquelle ladite caractéristique de dissolution est que moins de 65% du zonisamide dans les billes de zonisamide à libération prolongée sont dissous dans la première heure lors d'un essai de dissolution type.

8. Formulation pharmaceutique selon la revendication 6, dans laquelle ladite caractéristique de dissolution est que moins de 50% du zonisamide dans les billes de zonisamide à libération prolongée sont dissous dans la première heure lors d'un essai de dissolution type.

9. Formulation pharmaceutique selon l'une quelconque des revendications 1 à 8, dans laquelle ledit excipient retard comprend au moins un élément choisi dans le groupe constitué par l'hydroxypropylméthylcellulose (HPMC), l'hydroxyéthylcellulose, l'hydroxypropylcellulose (HPC), la méthylcellulose, l'éthylcellulose, l'acétate butyrate de cellulose, l'acétate phtalate de cellulose, le phtalate d'hydroxypropylméthylcellulose, la cellulose microcristalline, l'amidon de maïs, l'oxyde de polyéthylène, l'alcool polyvinylique (PVA), la polyvinylpyrrolidone (PVP), la PVP réticulée, l'acétate phtalate de polyvinyle, le polyéthylène glycol, la zéine, le poly(DL-lactide-co-glycolide), le phosphate dicalcique, le sulfate de calcium, et les mélanges de ceux-ci.

10. Formulation pharmaceutique selon l'une quelconque des revendications 1 à 9, dans laquelle ladite formulation pharmaceutique comprend au moins 10% en masse dudit excipient retard.

11. Formulation pharmaceutique selon l'une quelconque des revendications 1 à 10, dans laquelle ledit excipient retard est configuré pour fournir, lors de l'administration à un patient, une valeur moyenne Cₘₐₓ de zonisamide sérique libre qui est inférieure à une valeur moyenne Cₘₐₓ de zonisamide sérique libre d'un zonisamide à libération immédiate comparable dans des conditions comparables.

12. Formulation pharmaceutique selon l'une quelconque des revendications 1 à 10, dans laquelle ledit excipient retard est configuré pour fournir, lors de l'administration à un patient, une valeur moyenne Cₘₐₓ de zonisamide sérique libre qui est d'au moins 5% inférieure à une valeur moyenne Cₘₐₓ de zonisamide sérique libre d'un zonisamide à libération immédiate comparable dans des conditions comparables.

13. Formulation pharmaceutique selon l'une quelconque des revendications 1 à 12, comprenant en outre du bupropion.

14. Formulation pharmaceutique selon l'une quelconque des revendications 1 à 13, configurée dans une forme posologique choisie parmi deux fois par jour, une fois par jour, une fois tous les deux jours, une fois tous les trois jours, une fois tous les quatre jours, une fois tous les cinq jours, une fois tous les six jours et une fois par semaine.

15. Utilisation d'une formulation pharmaceutique selon l'une quelconque des revendications 1 à 14, pour la préparation d'un médicament destiné à affecter la perte de poids, à augmenter la dépense d'énergie, à augmenter la satiété ou à supprimer l'appétit.

16. Utilisation d'une formulation pharmaceutique selon l'une quelconque des revendications 1 à 14, pour la préparation d'un médicament destiné à réduire le risque d'un événement indésirable associé à l'administration d'une posologie comparable d'un zonisamide à libération immédiate.
